# EUROPÄISCHE PATENTSCHRIFT

(11) **EP 3 377 609 B1**
(45) Veröffentlichungstag und Bekanntmachung des Hinweises auf die Patenterteilung: **15.01.2020**
(21) Anmeldenummer: 16801727.5
(22) Anmeldetag: 16.11.2016
(51) Int. Cl.: C12M 1/34

(54) **MISCHVORRICHTUNG ZUM DURCHMISCHEN EINES BIOREAKTORS AUFWEISEND EINE SYNCHRONISIERUNGSEINRICHTUNG**
MIXING DEVICE FOR MIXING THE CONTENT OF A BIOREACTOR, COMPRISING A SYNCHRONIZATION DEVICE
DISPOSITIF DE MÉLANGE POUR LE BRASSAGE D'UN BIORÉACTEUR POSSÉDANT UN DISPOSITIF DE SYNCHRONISATION

(30) Priorität: 16.11.2015 DE 102015119756
(43) Veröffentlichungstag der Anmeldung: 26.09.2018
(73) Patentinhaber: Sartorius Stedim Biotech GmbH, 37079 Göttingen (DE)
(72) Erfinder: BRÜTSCH, Simon, 8600 Dübendorf (CH); SCHOLZ, Jochen, 37077 Göttingen (DE)
(74) Vertreter: Michalski Hüttermann & Partner Patentanwälte mbB
(86) Internationale Anmeldenummer: PCT/EP2016/077880
(87) Internationale Veröffentlichungsnummer: WO 2017/085136

(56) Entgegenhaltungen:
- WO-A1-2006/017951
- CH-A5- 697 035
- DE-A1- 3 248 543
- DE-A1-102004 039 463
- DE-A1-102009 037 345
- DE-A1-102011 017 781
- DE-A1-102014 105 472
- DE-B3-102009 055 406
- JP-A- 2005 261 230

## Beschreibung

Die vorliegende Erfindung betrifft eine Mischvorrichtung zum Durchmischen eines Bioreaktors gemäß dem Oberbegriff des unabhängigen Anspruchs, sowie ein Verfahren zur Durchmischung eines Bioreaktors.

Bei der Herstellung von Kulturmedien für Mikroorganismen bzw. Zellkulturen oder bei kontrollierten biotechnologischen Prozessen wie der Zellkultivierung kommt der Durchmischung bzw. dem Umwälzen des Bioreaktors eine besondere Bedeutung zu. Aufgrund der unterschiedlichen Ausgangskonzentrationen der Stoffe sowie der Stoffwechselaktivität der Mikroorganismen bzw. Zellen während der Kultivierung kommt es zu lokalen Konzentrationsänderungen verschiedenster chemischer Komponenten im Medienansatz oder von Nährstoffen, Sauerstoff und den entstehenden Metaboliten während der Kultivierung. Um im gesamten Behältnis gleiche oder zumindest kontrollierte Konzentrationsbedingungen zu gewährleisten, ist es erforderlich, die Flüssigkeit oder Suspension im Behälter während des gesamten Prozesses durchzumischen bzw. umzuwälzen.

Der Einsatz von flexiblen Einwegbioreaktoren aus Folien nimmt dabei insbesondere im Hinblick auf die ständig steigenden Anforderungen an die Sterilität der Prozesse in der Bioverfahrenstechnik gegenüber starren Behältern aus Glas oder Edelstahl stetig an Bedeutung zu. Neben der guten Sterilisierbarkeit bieten die Folienbeutel weitere Vorteile wie die kostengünstige Herstellung, die einfache und platzsparende Lagerung, die Sicherheit gegenüber Kontamination und den Entfall einer aufwendigen Reinigung nach dem Gebrauch. Vorrichtungen zum Durchmischen derartiger Bioreaktoren sind auch als Rocker, Plattformschüttler, Taumelmischer, Kreisschüttler, Vibrationsschüttler, Horizontalschüttler, Orbital-Shaker bekannt, und beispielsweise in den Offenlegungsschriften DE3248543A1, CH697035A5 oder EP1778828B1 beschrieben.

Solche Mischer werden beispielsweise von der Firma Sartorius unter der Marke BIOSTAT® RM vertrieben. Dabei wird der gesamte Bioreaktor so bewegt, dass die Bewegung zu einer Durchmischung des Reaktorinhalts führt.

Häufig verfügen diese Vorrichtungen über Sensoren zur Überwachung und Kontrolle des Kultivierungsprozesses. Diese können innerhalb und außerhalb des Bioreaktors angeordnet sein.

JP20052612309 offenbart eine Schüttelvorrichtung mit einem Zellkulturbehälter, eine von einer Antriebselle angetriebene Exzenterwelle und einer Hilfsexzenterwelle die synchron zur Drehung der Hauptexzenterwelle drehbar angetrieben wird. Die Vorrichtung verfügt über Motorstromsensoren und einen Sensor zum Erfassen der Drehzahl des Motors.

DE102009055406 offenbart eine Vorrichtung zur Aufnahme von Messdaten mit einem Aktivierungselement (106) zur Aktivierung oder Desaktivierung eines Messvorgangs. Mittels eines im Aktivierungselement enthaltenen Sensors (107) lässt sich ein Messvorgang aktivieren.

DE102004039463 offenbart einen Sensor mit einem Sensorelement und einer Kalibrierungsvorrichtung.

Dabei kann es vorkommen, dass das Sensorsignal von dem schwankenden Flüssigkeitsniveau innerhalb des Bioreaktors beeinträchtigt wird, was zu hohen Signalschwankungen oder Fehlmessungen führen kann.

Eine solche Störung kann beispielsweise dadurch hervorgerufen werden, dass bei einem innerhalb des Bioreaktors angeordneten Sensor das Sensorsignal von der zeitlich variierenden Flüssigkeitssäule beeinflusst wird. So kann der Sensor zeitweise freiliegen und zeitweise von Flüssigkeit bedeckt sein, oder die auf ihm lastende Flüssigkeitssäule kann Höhenschwankungen unterliegen.

Dies trifft beispielsweise für Impedanzsensoren zu, mit deren Hilfe beispielsweise die Biomasse der lebenden Zellen in einer Kulturflüssigkeit ermittelt werden kann.

Ebenso trifft dies beispielsweise für Leitfähigkeitssensoren zu, mit deren Hilfe die Leitfähigkeit und somit ein Maß für die gelösten Stoffe in der Zellsuspension ermittelt werden kann.

In beiden Fällen ist ein reproduzierbares Messergebnis nur dann erzeugbar, wenn besagte Sensoren von einer idealerweise stets gleich hohen Flüssigkeitssäule bedeckt sind bzw. eine Mindestbedeckung vorliegt.

Aufgabe der vorliegenden Erfindung ist es daher, eine Mischvorrichtung zum Durchmischen eines Bioreaktors bzw. ein Verfahren zum Durchmischen eines Bioreaktors bereitzustellen, die bzw. das die oben genannten Nachteile nicht aufweist.

Es ist eine weitere Aufgabe der vorliegenden Erfindung, eine Mischvorrichtung zum Durchmischen eines Bioreaktors bzw. ein Verfahren zum Durchmischen eines Bioreaktors bereitzustellen die bzw. das eine reproduzierbare Überwachung und Kontrolle des Kultivierungsprozesses ermöglicht.

Diese und andere Aufgaben werden durch die Verfahren bzw. Vorrichtungen gemäß der unabhängigen Ansprüche der aktuellen Erfindung erfüllt. Die abhängigen Ansprüche beschreiben bevorzugte Ausführungsformen. Wertebereiche die durch numerische Werte begrenzt sind, sollen stets die besagten Grenzwerte beinhalten.

### Zusammenfassung der Erfindung

Bevor die Erfindung im Detail beschrieben wird, wird darauf hingewiesen, dass diese Erfindung nicht begrenzt ist auf bestimmte Bestandteile der beschriebenen Vorrichtungen, oder beschrieben Herstellungsschritte der Verfahren, da diese Verfahren bzw. Vorrichtungen variieren können. Es wird auch darauf hingewiesen, dass die Terminologie hierfür nur zum Zweck für bestimmte beschriebene Ausführungsformen benutzt wird, und nicht absichtlich begrenzt ist.

Es soll angemerkt werden, dass in der verwendeten Beschreibung und in den anhängenden Ansprüchen, die einfache Form wie "ein/eine" oder "der/die/das" einen singulären und/oder pluralen Gegenstand beinhaltet, außer wenn es im Kontext klar anders formuliert ist. Für den Fall, dass ein Parameterbereich angegeben wurde, zählen die begrenzenden Zahlenwerte als Grenzwerte zum offenbarten bzw. beanspruchten Zahlenbereich dazu.

Erfindungsgemäß ist ein System aufweisend eine Mischvorrichtung zum Durchmischen eines Bioreaktors vorgesehen, aufweisend mindestens eine Bewegungseinrichtung zur Einleitung einer Durchmischungs-Bewegung in den Bioreaktor oder in eine Halterung zur Aufnahme eines Bioreaktors, mindestens einen in oder an dem Bioreaktor anordenbaren Sensor zur Aufnahme mindestens einer physiologischen oder physikalischen Messgröße, wobei die Mischvorrichtung oder der Bioreaktor ferner einen Sensor oder Geber zur Erzeugung einer Synchronisierungs-Messgröße aufweist.

Es ist dabei vorgesehen, dass das System ferner eine Synchronisierungseinrichtung zur
a) Auslösung der Messung mindestens einer physiologischen oder physikalischen Messgröße auf Basis der Synchronisierung-Messgröße, und ggf. anschließender Auswertung mindestens eines gemessenen Werts, oder
b) Auswahl mindestens eines aufgezeichneten Werts mindestens einer physiologischen oder physikalischen Messgröße auf Basis der Synchronisierung-Messgröße, und ggf. anschließender Auswertung des mindestens einen Werts, oder
c) Verrechnung der Synchronisierungs-Messgröße mit der mindestens einen physiologischen oder physikalischen Messgröße
aufweist.

Bevorzugt ist dabei weiterhin vorgesehen, dass das System eine Auswerteeinrichtung zur Darstellung, Aufzeichnung und/oder Auswertung der mindestens einen physiologischen oder physikalischen Messgröße aufweist.

Vorrichtungen zum Durchmischen derartiger weisen in der Regel eine plattenförmige oder wannenförmige Halterung für einen Einwegreaktor, beispielsweise einen Bioreaktor, auf.

Die Bioreaktoren werden auf besagter Halterung angeordnet, wobei sie z.B. mittels Klemmen oder Haken oder sonstigen lösbaren Befestigungsmitteln fixiert werden. Die Halterung wird angetrieben und erzeugt die Mischbewegung des Inhalts des Bioreaktors. Die Halterung führt hierbei beispielsweise eine Wippbewegung um eine horizontale Lage aus. Die Bioreaktoren können Volumina zwischen 0,5 und 1000 L aufweisen.

Es ist ist vorgesehen, dass die Synchronisierungs-Messgröße mit der von der Bewegungseinrichtung Bewegung oder der Durchmischungsbewegung im Bioreaktor synchronisiert oder synchronisierbar ist.

Der Begriff "synchronisiert" oder "synchronisierbar", wie hier verwendet, umfasst insbesodere all solche Ansätze, bei denen die Synchronisierungs-Messgröße so ausgewählt ist, dass sie einen Takt oder Rhythmus der Durchmischungs-Bewegung aufnimmt oder einen generierten Takt oder Rhythmus eines durch die Durchmischungs-Bewegung direkt oder indirekt generierten Artefakts aufnimmt.

Es ist ist vorgesehen, dass der Sensor zur Aufnahme einer physiologischen oder physikalischen Messgröße und der Sensor oder Geber zur Erzeugung einer Synchronisierungs-Messgröße identisch sind.

In einer solchen Ausführungsform kann beispielsweise die physiologische oder physikalische Messgröße diskontinuierlich aufgenommen werden, beispielsweise mit einer bestimmten Samplingfrequenz. Über einen Auswertealgorithmus kann dann beispielsweise bewerkstelligt werden, dass immer dann, wenn die physiologische oder physikalische Messgröße einen bestimmten Schwellenwert über- oder unterschreitet, der folgende oder vorhergehende Messwert herangezogen wird.

Eine solche Ausführungsform lässt sich beispielsweise im Vorfeld eichen oder Teachen, indem der Bediener mit unterschiedlichen Schwellenwerten experimentiert.

In einer anderen bevorzugten Ausführungsform ist vorgesehen, dass ein Sensor zur Aufnahme einer physiologischen oder physikalischen Messgröße und ein Sensor oder Gebers zur Erzeugung einer Synchronisierungs-Messgröße verschieden voneinander sind.

Bevorzugt ist ferner vorgesehen, dass der Sensor zur Aufnahme einer physiologischen oder physikalischen Messgröße zur Aufnahme einer Zeitreihe einer physiologischen oder physikalischen Messgröße eingerichtet ist.

Bevorzugt ist weiterhin vorgesehen, dass der Sensor oder Geber zur Erzeugung einer Synchronisierungs-Messgröße zur Erzeugung einer Zeitreihe einer Synchronisierungs-Messgröße eingerichtet ist.

Bevorzugt ist weiterhin vorgesehen, dass der Bioreaktor auf einer Halterung angeordnet ist, in welche die Durchmischungs-Bewegung einleitbar ist.

Bevorzugt ist dabei das System so ausgebildet, dass es mindestens eine Durchmischungs-Bewegung ausgewählt aus der Gruppe enthaltend
- eine periodische Bewegung
- eine zweidimensionale Bewegung
- eine dreidimensionale Bewegung
- eine Kippbewegung um eine horiziontale Achse und/oder
- eine Drehbewegung um eine vertikale Achse.
in den Bioreaktor oder in die Halterung zur Aufnahme eines Bioreaktors einleiten kann.

Dabei liegen die Frequenzen der Durchmischungsbewegung bevorzugt im Bereich zwischen ≥ 5 und ≤ 200 min⁻¹ (also ≥ 0,083 und ≤ 3,33 Hz). Bei einer Kippbewegung liegt der Winkel bevorzugt im Bereich zwischen +/- 3° und +/- 15°.

Bevorzugt ist ferner vorgesehen, dass der Sensor zur Aufnahme mindestens einer physiologischen oder physikalischen Messgröße ausgewählt ist aus der Gruppe enthaltend
- Kapazitiver Sensor
- Temperatursensor
- Impedanzsensor
- Leitfähigkeitssensor
- Sensor zur Messung der optischen Dichte und/oder Trübung
- Sensor zur Messung von Streulicht
- Optischer Sensor zur Spektroskopie, insbesondere Absorptionsspektrokopie
- Fluoreszenzsensor
- Impedanzspektroskopiesensor, , und/oder
- FTIR-Spektroskop

Wichtig ist hierbei, dass die Datenaufnahme in Bezug auf die physiologische oder physikalische Messgröße je nach Sensor-Typ unterschiedlich lang dauern kann bzw. unterschiedlich lange Signalverarbeitungszeiten zwischen aufeinander folgenden Messungen benötigt werden, was sich auf die erreichbare Frequenz der Datenaufnahme auswirkt.

Sensoren, die mit einer hohen Frequenz auslesbar sind, eignen sich bevorzugt für eine kontinuierliche bzw. pseudokontinuierliche Datenaufnahme (d.h., mit einer hohen Sampling-Frequenz), während sich Sensoren, die nur mit einer niedrigen Frequenz auslesbar sind, für eine diskontinuierliche Datenaufnahme (d.h., z.B. ausgelöst durch die Synchronisierungsmessgröße) eignen.

Bevorzugt ist weiterhin vorgesehen, dass die aufzunehmende physiologische oder physikalische Messgröße ausgewählt ist aus der Gruppe enthaltend
- Biomasse
- Leitfähigkeit
- pH-Wert
- Temperatur
- pO₂ und/oder pCO₂
- optische Dichte
- Kapazität
- Zellparameter
- Zelldichte
- Zelldurchmesser, und/oder
- Metabolitenkonzentration.

Diese Messgrößen erlauben einzeln oder in Kombination eine Aussage über (i) den Kultivierungsprozess (Anwachsen der Zellmenge, Zellviabilität etc), (ii) die Qualität der Kultivierungsbedingungen sowie des Mediums und ähnliches.

Bevorzugt ist weiterhin vorgesehen, dass der Sensor oder Geber zur Erzeugung einer Synchronisierungs-Messgröße ausgewählt ist aus der Gruppe enthaltend
- Sensor oder Geber zur Erfassung einer Winkelposition der Bewegungseinrichtung
- Sensor oder Geber zur Erfassung von Daten eines Elektro-, Servo- oder Schrittmotors oder einer Elektro-, Servo- oder Schrittmotorsteuerung
- Näherungssensor zur Erfassung der Position des Bioreaktors bzw. seiner Halterung
- Sensor oder Geber zur Erfassung der Leistungsaufnahme eines Elektro-, Servo- oder Schrittmotors
- Beschleunigungssensor zur Erfassung der momentanen Bewegungsänderung des Bioreaktors bzw. seiner Halterung
- Füllstandsmesser zur Messung eines Flüssigkeitspegels in dem Bioreaktor
- Druck-, Last- oder Wägesensor, und/oder
- Kamerasystem/digitale Bildverarbeitung

Auch hier gilt, dass Sensoren, die mit einer hohen Frequenz auslesbar sind, sich bevorzugt für eine kontinuierliche bzw. pseudokontinuierliche Erzeugung der Synchronisierungs-Messgröße eignen (d.h., mit einer hohen Sampling-Frequenz), während sich Sensoren, die nur mit einer niedrigen Frequenz auslesbar sind, für eine diskontinuierliche Erzeugung der Synchronisierungs-Messgröße eignen (z.B. durch Betätigen eines Schalters o.ä.).

Wichtig ist in diesem Zusammenhang auch, dass der im Falle der
a) Auslösung der Messung mindestens einer physiologischen oder physikalischen Messgröße auf Basis der Synchronisierung-Messgröße, bzw.
b) Auswahl mindestens eines aufgezeichneten Werts mindestens einer physiologischen oder physikalischen Messgröße auf Basis der Synchronisierung-Messgröße, und ggf. anschließender Auswertung des mindestens einen Werts.
ggf. ein etwaiger zeitlicher Versatz berücksichtigt werden muss. So folgt beispielsweise die in dem Bioreaktor vorhandene Flüssigkeit der eingeleiteten Durchmischungsbewegung (beispielsweise einer Kippbewegung) mit einer Phasenverschiebung.

Gemäß der vorliegenden Erfindung erzeugt ein Geber zur Erzeugung einer Synchronisierungs-Messgröße ein Triggersignal oder ein digitales Signal (digital im Sinne von An/Aus oder High/Low). Hierzu kann der Geber beispielsweise einen Schalter aufweisen, der beispielsweise immer dann, wenn der Rocker bzw. Plattformschüttler in die maximale Auslenkung gekommen ist und seine Bewegungsrichtung umkehrt, betätigt wird. In dem Augenblick der Betätigung ist die durch die Bewegung ausgelöste Welle in der Kulturflüssigkeit im Bioreaktor jedoch noch nicht an dem betreffenden Ende des Behälters angekommen; sie tut dies mit einer gewissen Zeitverzögerung. In dem Augenblick der Betätigung ist daher eine über einem an dem betreffenden Ende des Behälters angeordneten Sensor stehende Flüssigkeitssäule noch nicht maximal - ihre Höhe folgt der Durchmischungsbewegung mit einer Phasenverschiebung.

Es kann daher sinnvoll sein, besagte Phasenverschiebung bei der Auslösung der Messung mindestens einer physiologischen oder physikalischen Messgröße bzw. der Auswahl mindestens eines aufgezeichneten Werts mindestens einer physiologischen oder physikalischen Messgröße zu berücksichtigen.

Bevorzugt ist ferner vorgesehen, dass die Synchronisierungseinrichtung Bestandteil der Auswerteeinrichtung ist.

Bevorzugt ist weiterhin vorgesehen, dass der Bioreaktor ein flexibler Einweg-Bioreaktor aus einem Folienmaterial ist.

Der Einsatz von flexiblen Einwegbioreaktoren aus Folien nimmt dabei insbesondere im Hinblick auf die ständig steigenden Anforderungen an die Sterilität der Prozesse in der Bioverfahrenstechnik gegenüber starren Behältern aus Glas oder Edelstahl stetig an Bedeutung zu. Neben der guten Sterilisierbarkeit bieten die Folienbeutel weitere Vorteile wie die kostengünstige Herstellung, die einfache und platzsparende Lagerung, die weitestgehende Sicherheit gegenüber Kontamination und den Entfall einer aufwendigen Reinigung nach dem Gebrauch.

Solche flexiblen Einwegbioreaktoren werden beispielsweise von der Firma Sartorius unter der Marke "CultiBags ® RM" und "Flexsafe ® RM" vertrieben. Sie sind beispielsweise in Größen von 2L, 10L, 20 L, 50L, 100L und 200 L erhältlich.

Erfindungsgemäß ist weiterhin eine Mischvorrichtung zum Durchmischen eines Bioreaktors vorgesehen, wobei besagte Mischvorrichtung eingerichtet ist zur Verwendung in einem System gemäß obiger Beschreibung. Die Mischvorrichtung weist mindestens eine Bewegungseinrichtung zur Einleitung einer Durchmischungs-Bewegung in den Bioreaktor oder in eine Halterung zur Aufnahme eines Bioreaktor auf.

Erfindungsgemäß ist weiterhin ein Verfahren zum Durchmischen eines Bioreaktors vorgesehen, wobei mittels einer Bewegungseinrichtung eine Durchmischungs-Bewegung in einen Bioreaktor eingeleitet wird, mittels eines in oder an dem Bioreaktor angeordneten Sensors mindestens eine physiologische oder physikalische Messgröße aufgenommen wird, mittels einer Auswerteeinrichtung die mindestens eine physiologische oder physikalische Messgröße dargestellt, aufgezeichnet und/oder ausgewertet wird, mittels eines Sensors oder Gebers zur Erzeugung einer Synchronisierungs-Messgröße eine Synchronisierungs-Messgröße erzeugt wird, und wobei mittels einer Synchronisierungseinrichtung
a) die Messung mindestens einer physiologischen oder physikalischen Messgröße ausgelöst und mindestens ein gemessener Wert anschließend ggf. angezeigt, aufgezeichnet und/oder ausgewertet wird, oder
b) aufgezeichnete Werte der mindestens einen physiologischen oder physikalischen Messgröße auf Basis der Synchronisierungs-Messgröße ausgewählt und ggf. anschließend ausgewertet werden, oder
c) die Synchronisierungs-Messgröße mit der mindestens einen physiologischen oder physikalischen Messgröße verrechnet wird.

In Bezug auf die erfindungsgemäße Vorrichtung und das erfindungsgemäße Verfahren ist also zu unterscheiden zwischen kontinuierlich (bzw. bei digitaler Datenaufnahme pseudokontinuierlich, also gesamplet) und diskontinuierlich aufgenommenen physiologischen oder physikalischen Messgröße und Synchronisierung-Messgrößen.

Beispielsweise kann die Synchronisierung-Messgröße kontinuierlich bzw. pseudokontinuierlich aufgenommen werden. Bei Erfüllung eines bestimmte Kriteriums, beispielsweise bei Über- oder Unterschreiten einer gegebenen Schwelle, Nulldurchgang Erreichen eines Lokalmaximums bzw. Lokalminimums kann dann die Messung einer physiologischen oder physikalischen Messgröße ausgelöst werden. In diesem Fall wird die physiologische oder physikalische Messgröße diskontinuierlich aufgenommen

Beispielsweise kann die Synchronisierung-Messgröße diskontinuierlich aufgenommen, beispielswiese durch Auslösen eines Schalters immer dann, wenn die Bewegungseinrichtung eine bestimmte Position durchfährt. Diese diskontinuierlich aufgenommene Synchronisierung-Messgröße kann dann die Messung einer physiologischen oder physikalischen Messgröße ausgelöst werden (diskontinuierlich) oder in einer kontinuierlich bzw. pseudokontinuierlich aufgenommenen physiologischen oder physikalischen Messgröße bestimmte Werte auswählen.

Ebenso kann die diskontinuierlich oder kontinuierlich bzw. pseudokontinuierlich aufgenommene Synchronisierungs-Messgröße mit der diskontinuierlich oder kontinuierlich bzw. pseudokontinuierlich aufgenommenen physiologischen oder physikalischen Messgröße verrechnet werden.

Erfindungsgemäß ist weiterhin ein Verfahren zur Ermittlung mindestens eines optimalen Messzeitpunkts oder Messzeitraums zur Aufnahme mindestens einer physiologischen oder physikalischen Messgröße mittels eines in oder an einem Bioreaktor angeordneten Sensors vorgesehen, wobei in den Bioreaktor mittels einer Bewegungseinrichtung eine Durchmischungs-Bewegung eingeleitet wird, wobei mittels eines Sensors oder Gebers eine Synchronisierungs-Messgröße erzeugt wird, und wobei mittels einer Synchronisierungseinrichtung
a) die Messung mindestens einer physiologischen oder physikalischen Messgröße ausgelöst und mindestens ein gemessener Wert anschließend ggf. angezeigt, aufgezeichnet und/oder ausgewertet wird, oder
b) die Synchronisierungs-Messgröße mit der mindestens einen physiologischen oder physikalischen Messgröße verrechnet wird, oder
c) aufgezeichnete Werte der mindestens einen physiologischen oder physikalischen Messgröße auf Basis der Synchronisierungs-Messgröße ausgewählt und ggf. anschlie-βend ausgewertet werden, oder
d) mindestens eine physiologische oder physikalische Messgröße auf Basis der Synchronisierungsmessgröße aufgenommen, nach einer vorgegebenen Regel in Abhängigkeit zur Synchronisierungsmessgröße ausgewählt und nachfolgend als Vorgabe für die weitere Aufnahme der mindestens einen physiologischen oder physikalischen Messgröße verwendet wird.

Bevorzugt ist dabei die Durchmischungs-Bewegung mindestens eine ausgewählt aus der Gruppe enthaltend
- eine periodische Bewegung
- eine zweidimensionale Bewegung
- eine dreidimensionale Bewegung
- eine Kippbewegung um eine horiziontale Achse und/oder
- eine Drehbewegung um eine vertikale Achse.

In einer besonders bevorzugten Ausführungsform ist vorgesehen, dass zur Aufnahme der physiologischen oder physikalischen Messgröße ein Frequenzspektrum durchfahren wird, und/oder die physiologische oder physikalische Messgröße aus einem Frequenzspektrum besteht.

Einige Messverfahren benutzen eine modulierte Messgröße, die in ihrer Frequenz dynamisch verändert wird. Dabei wird für jede Messung ein Frequenzspektrum durchfahren (ein sogenannter Sweep). Letzteres kommt bei vielen Spektroskopischen Verfahren zum Einsatz, beispielsweise in der Impedanzspektroskopie sowie optischen Spektroskopieverfahren wie der UV-, IR-, oder Raman-Spektroskopie und FTIR. Gerade derart erzeugte physiologische oder physikalischen Messgrößen müssen in der Regel diskontinuierlich aufgenommen werden.

Dabei kann es vorkommen, dass die Auslesefrequenz betreffend die physiologische oder physikalische Messgröße geringer oder in vergleichbarer Größe ist wie die Frequenz der Durchmischungsbewegung. Mit anderen Worten: Der für das Durchfahren des Frequenzspektrums ("Sweepdauer") benötigte Zeitraum kann gegenüber der Änderung der Flüssigkeitshöhe im Reaktor nicht vernachlässigt werden.

Besonders bevorzugt ist dabei vorgesehen, dass das Frequenzspektrum in Abhängigkeit der Synchronisierungs-Messgröße diskret und diskontinuierlich aufgenommen wird.

So kann sichergestellt werden, dass die verschiedenen Frequenzen bzw. Wellenlängen des Spektrums jeweils zu einem Zeitpunkt gemessen werden, bei welchem der Flüssigkeitsstand an der Messposition identisch ist, so dass ein reproduzierbares und von durch Flüssigkeitsschwankungen unbeeinflusstes Spektrum aufgenommen werden kann.

Erfindungsgemäß ist weiterhin ein System oder eine Vorrichtung gemäß obiger Beschreibung zur Verwendung in einem Verfahren gemäß obiger Beschreibung vorgesehen.

Erfindungsgemäß ist weiterhin die Verwendung eines Systems, einer Vorrichung oder eines Verfahrens gemäß obiger Beschreibung für die Kultivierung von Mikroorganismen und/oder Zellen vorgesehen.

Hierbei kommt insbesondere die Kultivierung von Säugetierzellen, Pflanzenzellen, Insektenzellen, Stammzellen und Mikroorganismen, infrage.

Bevorzugt erfolgt die Kultivierung besagter Mikroorganismen und/oder Zellen zur Vermehrung derselben und/oder zur Produktion von Biomolekülen, wie Proteinen, insbesondere zu industriellen oder pharmazeutischen Zwecken.

Bei besagten Proteinen kann es sich beispielsweise um Antikörper, Hormone, Enzyme, Wachstumsfaktoren, Cytokine und dergleichen handeln.

Weiterhin ist erfindungsgemäß ein Verfahren zum Teachen oder Eichen eines Systems gemäß obiger Beschreibung vorgesehen, wobei in einem Testdurchlauf ein Schwellenwert betreffend (a) die physiologische oder physikalische Messgröße oder (b) die Synchronisierungsmessgröße definiert oder ermittelt wird, bei deren Über- oder Unterschreitung, ein mit der über- oder Unterschreitung im zeitlichen Zusammenhang stehender Messwert der physiologischen oder physikalischen Messgröße herangezogen wird.

Auf diese Weise wird also in einem vorgelagerten Verfahren der bestgeeignete messzeitpunkt bestimmt.

### Zeichnungen und Beispiele

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken.

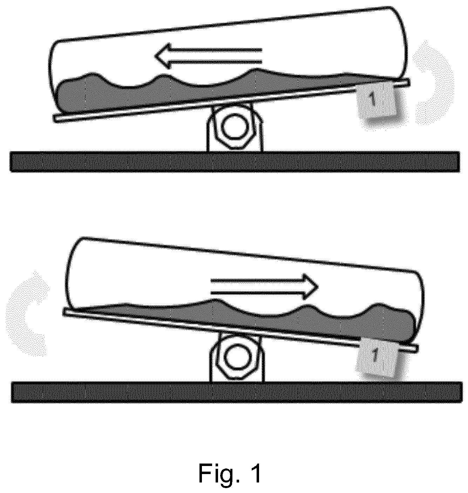

Fig. 1 zeigt einen Bioreaktor auf einem Rocker gemäß obiger Beschreibung mit einem Sensor 1 zur Aufnahme einer physiologischen oder physikalischen Messgröße. Erkennbar ist, dass über dem Sensor in Abhängigkeit von der Durchmischungsbewegung zeitweise eine hohe und zeitweise eine geringe Flüssigkeitssäule steht, die das Messergebnis des Sensors 1 beeinträchtigen kann.

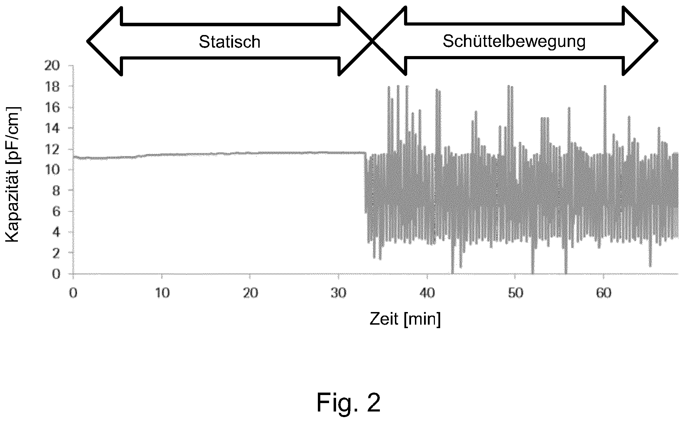

Fig. 2 zeigt eine Zeitreihe einer Kapazitätsmessung mit einem 4-Elektroden Sensor in einem Bioreaktor. In den ersten 35 Sekunden ruht der Bioreaktor, während ab der 35. Sekunde eine Durchmischungsbewegung in den Bioreaktor eingeleitet wird. Die dadurch erzeugte Wellenbewegung beeinträchtigt das Messsignal erheblich.

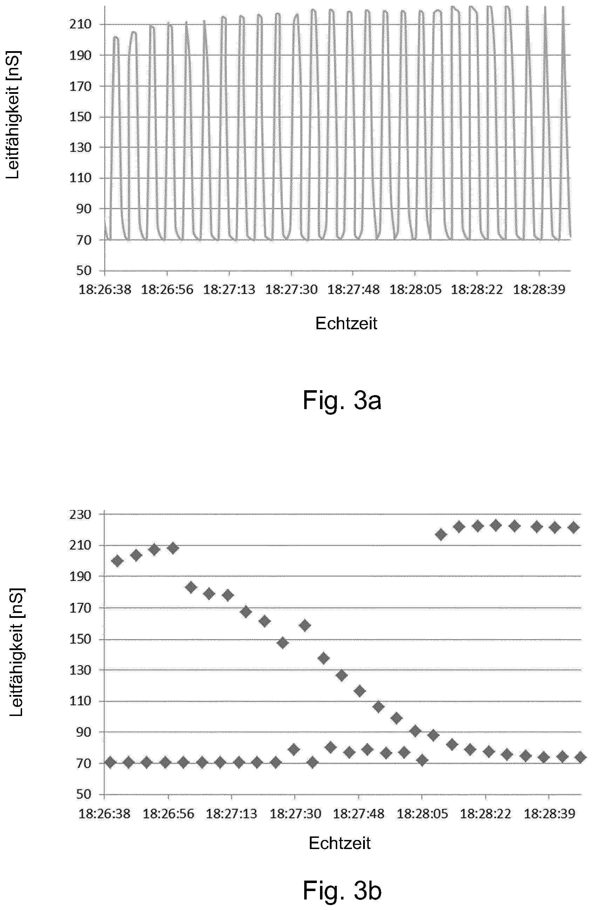

Fig. 3a zeigt eine physiologische Messgröße, hier die Leitfähigkeit, aus einem Bioreaktor, der auf einer erfindungsgemäßen Mischvorrichtung mit einer Kippbewegung um eine horizontale Achse mit einer Frequenz von 12 BPM bewegt wird. Durch die Wellenbewegung schwankt die Flüssigkeitssäule über dem Leitfähigkeitssensor, wodurch das Messsignal stark beeinflusst wird. Der Leitfähigkeitssensor zeigt die Leitfähigkeit zwischen den Messelektroden auf der Sensoroberfläche. Nur bei voller Bedeckung und ausreichender Flüssigkeitshöhe über dem Sensor entspricht das Messsignal somit der tatsächlichen Leitfähigkeit der Flüssigkeit im Sinne einer physiologischen Größe oder Stoffeigenschaft, die wiederrum Rückschlüsse auf die Lösungskonzentration eines gelösten Stoffs ermöglicht. In Fig. 3a sind also nur die Werte in den Maxima repräsentativ für die Leitfähigkeit als Kenngröße der im Bioreaktor befindlichen Flüssigkeit.

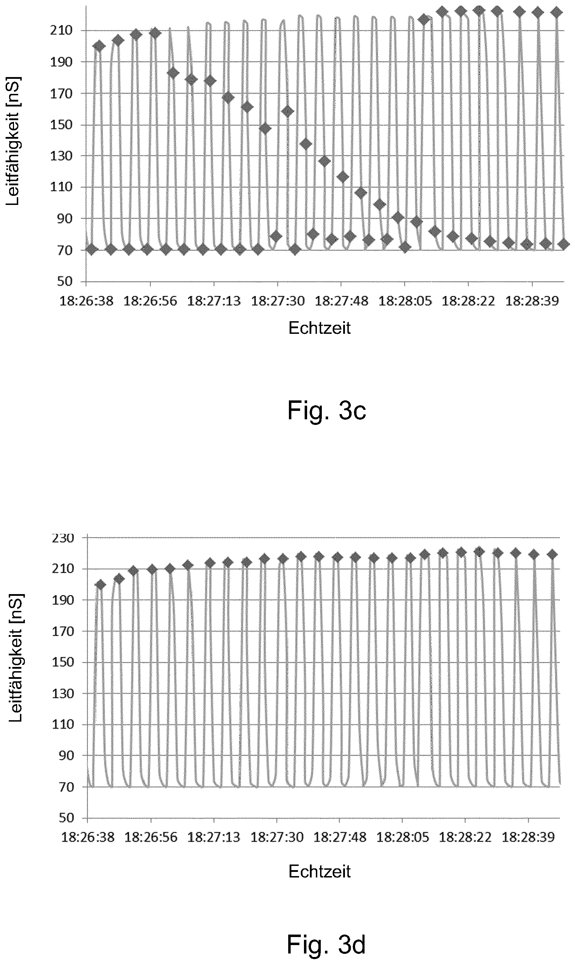

Setzt man in diesem System einen langsameren Leitfähigkeitsdetektor ein, der z.B. nur alle 3 Sekunden Werte liefert, so kommt man zu einem Messsignal, wie es die Messpunkte der Datenreihe in Fig. 3b darstellen: Teilweise werden noch die Maxima und Minima der durch die Flüssigkeitsbewegung modulierten Leitfähigkeit am Messort getroffen. Durch Schwebungseffekte wandern aber auch viele Messpunkte auf die Flanken. In dieser Messsituation ist es äußerst schwierig, den momentanen Maximalwert der Leitfähigkeit am Sensor, d.h. die Leitfähigkeit der Flüssigkeit als physiologische Größe, zu ermitteln. Dies wird in der Überlagerung von tatsächlicher Leitfähigkeit (durchgezogene Kurve) mit dem Sensorsignal des langsamen Sensors (Messpunkte) in Fig. 3c deutlich.

Wird jedoch der langsame Sensor, der zwischen zwei Messungen mindestens 3 Sekunden braucht, also eine Messfrequenz von maximal 0.333 Hz aufweist, mit der Wellenbewegung synchronisiert, so kann die Messung immer im relevanten Zeitpunkt der maximalen Sensorbedeckung ausgelöst werden. Diese Situation ist in Fig. 3d mit den Messpunkten dargestellt - hier wieder zum Vergleich überlagert mit der tatsächlichen modulierten Leitfähigkeit am Messort (durchgezogene Kurve). Der langsame Sensor misst hier nur mit ca. 0,2 Hz, aber aufgrund der Synchronisation mit der Wellenbewegung im Bioreaktor immer zum richtigen Zeitpunkt, wobei die Synchronisation z.B. mithilfe eines Füllstandsensors erfolgt. Der Sensor misst dabei nicht zwangsläufig mit einer festen Frequenz, da die Wellenbewegung teilweise chaotisch verläuft und die Synchronisationsmessgröße somit auch nur im Mittel der Periodizität der treibenden Bewegungseinrichtung entspricht.

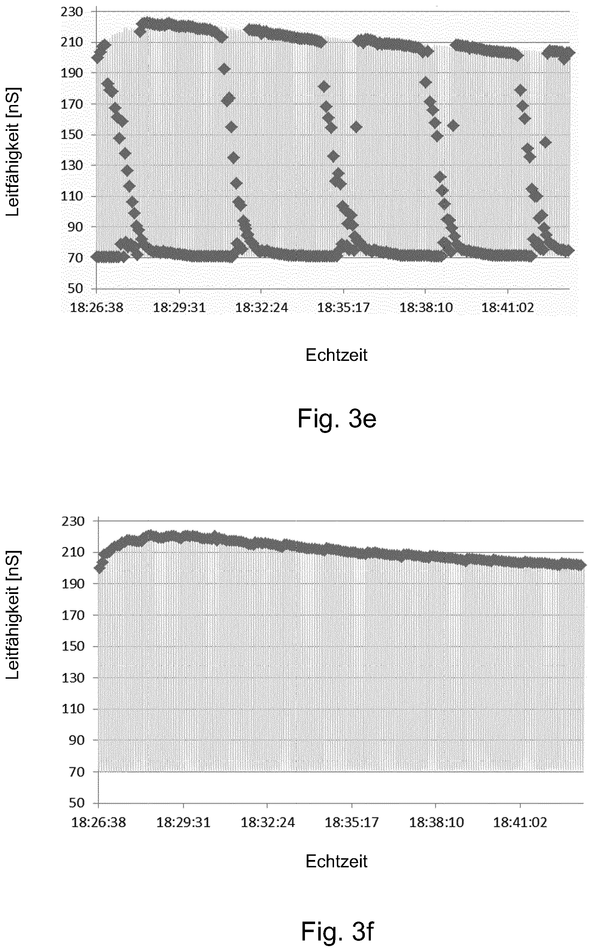

Der Vorteil der Synchronisation wird insbesondere deutlich, wenn sich die physiologische Messgröße, d.h. die Leitfähigkeit, zeitlich ändert und die Änderungen in ähnlichen Zeiträumen stattfinden, wie die Schwebungserscheinungen zwischen langsamer Sensordetektion und Durchmischungsbewegung. Ein solches Beispiel ist in Fig. 3e und 3f zusammengefasst. Wieder zeigen die durchgezogenen Kurven die jeweilige momentane Leitfähigkeit am Sensorort. Diese ist aufgrund der schwankenden Flüssigkeitshöhe über dem Sensor, verursacht durch die Durchmischungsbewegung, stark moduliert und entspricht nur in den Maximalwerten der zu messenden Leitfähigkeit der Flüssigkeit als physiologische Größe. Im Beispiel in Figs. 3e und 3f steigt die Leitfähigkeit der Flüssigkeit zunächst in einem Zeitraum von ca. 2 Minuten von 195nS auf 225nS an um dann über einen Zeitraum von ca. 12 Minuten wieder abzufallen. In Fig. 3e ist mit den Messpunkten wieder das entsprechende Messsignal eines langsamen Sensors mit 0,333Hz Messfrequenz mit aufgetragen. Insbesondere der Verlauf des zweiminütigen Signalanstiegs ist aus diesem Messsignal nicht mehr zu rekonstruieren. Fig. 3f zeigt hingegen das Messergebnis eines synchronisierten langsamen Detektors (Messpunkte) überlagert mit der tatsächlichen momentanen Leitfähigkeit. Als Synchronisierungsmessgröße dient hier z.B. wieder ein Füllstandsignal. In Fig. 3f kann somit der gesamte Vorgang der dynamischen Veränderung der physiologischen Größe verfolgt werden, da der langsame synchronisierte Sensor immer im relevanten Zeitpunkt der maximalen Sensorbedeckung misst.

## Patentansprüche

1. Ein eine Mischvorrichtung zum Durchmischen eines Bioreaktors aufweisendes System, wobei die Mischvorrichtung aufweist:
mindestens eine Bewegungseinrichtung zur Einleitung einer Durchmischungs-Bewegung in den Bioreaktor oder in eine Halterung zur Aufnahme eines Bioreaktors
mindestens einen in oder an dem Bioreaktor anordenbaren Sensor zur Aufnahme mindestens einer physiologischen oder physikalischen Messgröße,
wobei die Mischvorrichtung oder der Bioreaktor weiterhin einen Sensor oder Geber zur Erzeugung einer Synchronisierungs-Messgröße, nämlich eines Triggersignals oder eines digitalen Signals (An/Aus oder High/Low) aufweist,
wobei das System weiterhin eine Synchronisierungseinrichtung aufweist zur
a) Auslösung der Messung mindestens einer physiologischen oder physikalischen Messgröße auf Basis der Synchronisierung-Messgröße, und ggf. anschließender Auswertung mindestens eines gemessenen Werts, oder
b) Auswahl mindestens eines aufgezeichneten Werts mindestens einer physiologischen oder physikalischen Messgröße auf Basis der Synchronisierung-Messgröße, und ggf. anschließender Auswertung des mindestens einen Werts, oder
c) Verrechnung der Synchronisierungs-Messgröße mit der mindestens einen physiologischen oder physikalischen Messgröße.

2. System gemäß Anspruch 1, weiterhin aufweisend eine Auswerteeinrichtung zur Darstellung, Aufzeichnung und/oder Auswertung der mindestens einen physiologischen oder physikalischen Messgröße.

3. System gemäß einem der vorherigen Ansprüche, wobei die Synchronisierungs-Messgröße mit der von der Bewegungseinrichtung generierten Bewegung oder der Durchmischungsbewegung im Bioreaktor synchronisiert oder synchronisierbar ist.

4. System gemäß einem der vorherigen Ansprüche, wobei ein Sensor zur Aufnahme einer physiologischen oder physikalischen Messgröße und ein Sensor oder Gebers zur Erzeugung einer Synchronisierungs-Messgröße verschieden voneinander sind.

5. System gemäß einem der vorherigen Ansprüche, wobei der Sensor zur Aufnahme mindestens einer physiologischen oder physikalischen Messgröße ausgewählt ist aus der Gruppe enthaltend
• Kapazitiver Sensor
• Temperatursensor
• Impedanzsensor
• Leitfähigkeitssensor
• Sensor zur Messung der optischen Dichte und/oder Trübung
• Sensor zur Messung von Streulicht
• Optischer Sensor zur Spektroskopie, insbesondere Absorptionsspektrokopie
• Fluoreszenzsensor
• Impedanzspektroskopiesensor, und/oder
• FTIR-Spektroskop.

6. System gemäß einem der vorherigen Ansprüche, wobei die aufzunehmende physiologische oder physikalische Messgröße ausgewählt ist aus der Gruppe enthaltend
• Biomasse
• Leitfähigkeit
• pH-Wert
• Temperatur
• pO₂ und/oder pCO₂
• optische Dichte
• Kapazität
• Zellparameter
• Zelldichte
• Zelldurchmesser, und/oder
• Metabolitenkonzentration.

7. System gemäß einem der vorherigen Ansprüche, wobei der Sensor oder Geber zur Erzeugung einer Synchronisierungs-Messgröße ausgewählt ist aus der Gruppe enthaltend
• Sensor oder Geber zur Erfassung einer Winkelposition der Bewegungseinrichtung
• Sensor oder Geber zur Erfassung von Daten eines Elektro-, Servo- oder Schrittmotors oder einer Elektro-, Servo- oder Schrittmotorsteuerung
• Näherungssensor zur Erfassung der Position des Bioreaktors bzw. seiner Halterung
• Sensor oder Geber zur Erfassung der Leistungsaufnahme eines Elektro-, Servo- oder Schrittmotors
• Beschleunigungssensor zur Erfassung der momentanen Bewegungsänderung des Bioreaktors bzw. seiner Halterung
• Füllstandsmesser zur Messung eines Flüssigkeitspegels in dem Bioreaktor
• Druck-, Last- oder Wägesensor, und/oder
• Kamerasystem/digitale Bildverarbeitung.

8. System gemäß einem der Ansprüche 1 bis 7, wobei die Synchronisierungseinrichtung Bestandteil der Auswerteeinrichtung ist.

9. Verfahren zum Durchmischen eines Bioreaktors, aufweisend:
mittels einer Bewegungseinrichtung eine Durchmischungs-Bewegung in einen Bioreaktor einleiten,
mittels eines in oder an dem Bioreaktor angeordneten Sensors mindestens eine physiologische oder physikalische Messgröße aufnehmen,
mittels einer Auswerteeinrichtung, die mindestens eine physiologische oder physikalische Messgröße dargestellt, aufzeichnen und/oder auswerten,
mittels eines Sensors oder Gebers zur Erzeugung einer Synchronisierungs-Messgröße eine Synchronisierungs-Messgröße erzeugen,
und mittels einer Synchronisierungseinrichtung
a) die Messung mindestens einer physiologischen oder physikalischen Messgröße auslösen und mindestens einen gemessenen Wert anschließend ggf. anzeigen, aufzeichnen und/oder auswerten, oder
b) aufgezeichnete Werte der mindestens einen physiologischen oder physikalischen Messgröße auf Basis der Synchronisierungs-Messgröße auswählen und ggf. anschließend auswerten, oder
c) die Synchronisierungs-Messgröße mit der mindestens einen physiologischen oder physikalischen Messgröße verrechnen.

10. Verfahren zur Ermittlung mindestens eines optimalen Messzeitpunkts oder Messzeitraums zur Aufnahme mindestens einer physiologischen oder physikalischen Messgröße mittels eines in oder an einem Bioreaktor angeordneten Sensors, aufweisend:
in den Bioreaktor mittels einer Bewegungseinrichtung eine Durchmischungs-Bewegung einleiten, mittels eines Sensors oder Gebers zur Erzeugung einer Synchronisierungs-Messgröße eine Synchronisierungs-Messgröße erzeugen,
und mittels einer Synchronisierungseinrichtung
a) die Messung mindestens einer physiologischen oder physikalischen Messgröße auslösen und mindestens einen gemessenen Wert anschließend ggf. anzeigen, aufzeichnen und/oder auswerten,
b) die Synchronisierungs-Messgröße mit der mindestens einen physiologischen oder physikalischen Messgröße verrechnen, oder
c) aufgezeichnete Werte der mindestens einen physiologischen oder physikalischen Messgröße auf Basis der Synchronisierungs-Messgröße auswählen und ggf. anschließend auswerten, oder
d) mindestens eine physiologische oder physikalische Messgröße auf Basis der Synchronisierungsmessgröße aufnehmen, nach einer vorgegebenen Regel in Abhängigkeit zur Synchronisierungsmessgröße auswählen und nachfolgend als Vorgabe für die weitere Aufnahme der mindestens einen physiologischen oder physikalischen Messgröße verwenden.

11. Verfahren gemäß Anspruch 9 oder 10, wobei zur Aufnahme der physiologischen oder physikalischen Messgröße ein Frequenzspektrum durchfahren wird, und/oder die physiologische oder physikalische Messgröße aus einem Frequenzspektrum besteht.

12. Verfahren gemäß Anspruch 11, wobei das Frequenzspektrum in Abhängigkeit der Synchronisierungs-Messgröße diskret und diskontinuierlich aufgenommen wird.

13. Verwendung eines Systems gemäß einem der Ansprüche 1 - 8 zum Durchmischen eines Bioreaktors oder in einem Verfahren gemäß einem der Ansprüche 11 - 13.

14. Verwendung eines Systems bzw. eines Verfahrens gemäß einem der vorhergehenden Ansprüche für die Kultivierung von Mikroorganismen und/oder Zellen.

15. Verfahren zum Teachen oder Eichen eines Systems gemäß einem der der Ansprüche 1 - 8, weiterhin aufweisend in einem Testdurchlauf einen Schwellenwert betreffend (a) die physiologische oder physikalische Messgröße oder (b) die Synchronisierungsmessgröße definieren oder ermitteln, bei deren Über- oder Unterschreitung ein mit der Über- oder Unterschreitung im zeitlichen Zusammenhang stehender Messwert der physiologischen oder physikalischen Messgröße herangezogen wird.

## Claims

1. A system comprising a mixing device for mixing of a bioreactor, wherein the mixing device comprises:
at least one motion device for inducing a mixing motion in the bioreactor or into a holder for receiving a bioreactor,
at least one sensor arrangeable within or at the bioreactor for receiving at least one physiological or physical measurand,
wherein the mixing device or the bioreactor further comprises a sensor or transmitter for generating a synchronizing measurand, namely a trigger signal or a digital signal (on/off or high/low),
the system further comprising a synchronizing device for
a) triggering the measurement of at least one physiological or physical measurand based on the synchronisation measurand and optionally subsequent evaluation of at least one measured value, or
b) selection of at least one recorded value of at least one physiological or physical measurand on the basis of the synchronisation measurand, and optionally subsequent evaluation of the at least one value, or
c) setting-off the synchronization measurand with the at least one physiological or physical measurand.

2. The system according to claim 1, further comprising an evaluation device for displaying, recording and/or evaluating the at least one physiological or physical measurand.

3. The system according to any of the previous claims, wherein the synchronization measurand is synchronized or synchronizable with the movement generated by the movement device or the mixing movement in the bioreactor.

4. The system according to any of the preceding claims, wherein a sensor for recording a physiological or physical measurand and a sensor or transmitter for generating a synchronizing measurand are different from one another.

5. The system according to any of the preceding claims, wherein the sensor for recording at least one physiological or physical measurand is selected from the group comprising a
• capacitive sensor
• temperature sensor
• impedance sensor
• conductivity sensor
• sensor for measuring optical density and/or turbidity
• sensor for measuring scattered light
• optical sensor for spectroscopy, in particular absorption spectroscopy
• fluorescence sensor
• impedance spectroscopy sensor, and/or
• FTIR spectroscope.

6. The system according to any of the preceding claims, wherein the physiological or physical measurand to be recorded is selected from the group comprising
• biomass
• conductivity
• pH value
• temperature
• pO₂ and/or pCO₂
• optical density
• capacity
• cell parameter
• cell density
• cell diameter, and/or
• metabolite concentration.

7. The system according to any of the preceding claims, wherein the sensor or transmitter for generating a synchronizing measurand is selected from the group comprising a
• sensor or transmitter for detecting an angular position of the mixing device,
• sensor or transmitter for acquiring data from an electric, servo- or step motor or an electric, servo- or step motor controller,
• proximity sensor for detecting the position of the bioreactor or its holder,
• sensor or transmitter for recording the power consumption of an electric, servo- or step motor,
• acceleration sensor for detecting the current change in movement of the bioreactor or its holder,
• filling-level meter for measuring a liquid level in a bioreactor,
• pressure, load or weighing sensor, and/or
• camera system/digital image processing.

8. The system according to any of claims 1 to 7, wherein the synchronizing device is part of the evaluation device.

9. A method of mixing a bioreactor comprising:
initiating a mixing movement into a bioreactor by means of a movement device,
recording at least one physiological or physical measurand by means of a sensor arranged in or at the bioreactor,
recording and/or evaluating by means of an evaluation device which displays at least one physiological or physical measurand,
generating a synchronization measurand by means of a sensor or encoder for generating a synchronization measurand,
and by means of a synchronizing device
a) triggering the measurement of at least one physiological or physical measurand and, as applicable, subsequently displaying, recording and/or evaluating, at least one measured value, or
b) selecting recorded values of at least one physiological or physical measurand on the basis of the synchronization measurand and, as applicable, evaluating the same thereafter, or
c) off-setting the synchronization measurand with the at least one physiological or physical measurand.

10. A method for determining at least one optimal time point of measurement or time period of measurement for recording at least one physiological or physical measurand by means of a sensor arranged within or at a bioreactor, comprising:
introducing a mixing movement into the bioreactor by means of a movement device,
generating a synchronization measurand by means of a sensor or encoder for generating a synchronization measurand,
and by means of a synchronizing device
a) triggering the measurement of at least one physiological or physical measurand and subsequently displaying, recording and/or evaluating at least one measured value,
b) off-setting the synchronisation measurand with at least one physiological or physical measurand, or
c) selecting recorded values of at least one physiological or physical measurand on the basis of the synchronization measurand, and, as applicable, subsequently evaluating the same, or
d) recording at least one physiological or physical measurand on the basis of the synchronizing measurand, selecting the same according to a predetermined rule depending on the synchronizing measurand, and subsequently using it as a default for further recording the at least one physiological or physical measurand.

11. The method according to claim 9 or 10, wherein for recording the physiological or physical measurand a frequency spectrum is gone through, and/or the physiological or physical measurand consists of a frequency spectrum.

12. The method according to claim 11, wherein the frequency spectrum is recorded discretely and discontinuously depending on the synchronization measurand.

13. The use of a system according to any of claims 1-8 for mixing a bioreactor or in a method according to any of claims 11-13.

14. The use of a system or method according to any of the preceding claims for the cultivation of microorganisms and/or cells.

15. A method for teaching or calibrating a system according to any of claims 1 - 8, further comprising defining or determining in a test run a threshold value relating to (a) the physiological or physical measurand or (b) the synchronizing measurand, wherein upon exceeding or undershooting the same a measured value of the physiological or physical measurand is used that is temporally correlated to the exceeding or undershooting.

## Revendications

1. Système comportant un dispositif de mélange pour le brassage d'un bioréacteur,
dans lequel le dispositif de mélange comporte :
- au moins un dispositif de mouvement pour introduire un mouvement de brassage dans le bioréacteur ou dans un support destiné à recevoir un bioréacteur,
- au moins un capteur pouvant être agencé dans ou sur le bioréacteur afin d'acquérir au moins une grandeur de mesure physiologique ou physique,
dans lequel le dispositif de mélange ou le bioréacteur comporte en outre un capteur ou transmetteur afin de produire une grandeur de mesure de synchronisation, à savoir un signal de déclenchement ou un signal numérique (marche/arrêt ou High/Low),
dans lequel le système comporte en outre un dispositif de synchronisation afin de
a) déclencher la mesure d'au moins une grandeur de mesure physiologique ou physique sur la base de la grandeur de mesure de synchronisation, et le cas échéant évaluer ensuite au moins une valeur mesurée, ou
b) sélectionner au moins une valeur enregistrée d'au moins une grandeur de mesure physiologique ou physique sur la base de la grandeur de mesure de synchronisation, et le cas échéant évaluer l'au moins une valeur, ou
c) compenser la grandeur de mesure de synchronisation avec l'au moins une grandeur de mesure physiologique ou physique.

2. Système selon la revendication 1, comportant en outre un dispositif d'évaluation afin de représenter, enregistrer et/ou évaluer l'au moins une grandeur de mesure physiologique ou physique.

3. Système selon l'une quelconque des revendications précédentes, dans lequel la grandeur de mesure de synchronisation est ou peut être synchronisée avec le mouvement généré par le dispositif de mouvement ou le mouvement de brassage dans le bioréacteur.

4. Système selon l'une quelconque des revendications précédentes, dans lequel un capteur destiné à acquérir une grandeur de mesure physiologique ou physique et un capteur ou transmetteur destiné à produire une grandeur de mesure de synchronisation sont différents l'un de l'autre.

5. Système selon l'une quelconque des revendications précédentes, dans lequel le capteur destiné à acquérir au moins une grandeur de mesure physiologique ou physique est sélectionné dans le groupe contenant
- capteur capacitif
- capteur de température
- capteur d'impédance
- capteur de conductibilité
- capteur destiné à la mesure de la densité optique et/ou de la turbidité
- capteur destiné à la mesure de lumière diffusée
- capteur optique destiné à la spectroscopie, en particulier à la spectroscopie d'absorption
- capteur de fluorescence
- capteur de spectroscopie d'impédance, et/ou
- spectroscope FTIR.

6. Système selon l'une quelconque des revendications précédentes, dans lequel la grandeur de mesure physiologique ou physique à acquérir est sélectionnée dans le groupe contenant
- biomasse
- conductibilité
- valeur pH
- température
- pO₂ et/ou pCO₂
- densité optique
- capacité
- paramètre de cellule
- densité de cellule
- diamètre de cellule, et/ou
- concentration de métabolites.

7. Système selon l'une quelconque des revendications précédentes, dans lequel le capteur ou transmetteur destiné à produire une grandeur de mesure de synchronisation est sélectionné dans le groupe contenant
- capteur ou transmetteur destiné à la détection d'une position angulaire du dispositif de mouvement
- capteur ou transmetteur destiné à la détection de données d'un moteur électrique, d'un servomoteur ou d'un moteur pas à pas ou d'une commande de moteur électrique, de servomoteur ou de moteur pas à pas
- capteur de proximité destiné à la détection de la position du bioréacteur ou de son support
- capteur ou transmetteur destiné à la détection de la puissance absorbée d'un moteur électrique, d'un servomoteur ou d'un moteur pas à pas
- capteur d'accélération destiné à la détection de la variation de mouvement instantanée du bioréacteur ou de son support
- capteur de niveau destiné à la mesure d'un niveau de liquide dans le bioréacteur
- capteur de pression, de charge ou de poids, et/ou
- système de caméra / traitement numérique d'image.

8. Système selon l'une quelconque des revendications 1 à 7, dans lequel le dispositif de synchronisation fait partie du dispositif d'évaluation.

9. Procédé destiné au brassage d'un bioréacteur, comportant :
- au moyen d'un dispositif de mouvement, introduire un mouvement de brassage dans un bioréacteur,
- au moyen d'un capteur agencé dans ou sur le bioréacteur, acquérir au moins une grandeur de mesure physiologique ou physique,
- au moyen d'un dispositif d'évaluation, représenter, enregistrer et/ou évaluer l'au moins une grandeur de mesure physiologique ou physique,
- au moyen d'un capteur ou transmetteur destiné à produire une grandeur de mesure de synchronisation, produire une grandeur de mesure de synchronisation,
et, au moyen d'un dispositif de synchronisation,
a) déclencher la mesure d'au moins une grandeur de mesure physiologique ou physique et ensuite, le cas échéant, afficher, enregistrer et/ou évaluer au moins une valeur mesurée, ou
b) sélectionner des valeurs enregistrées de l'au moins une grandeur de mesure physiologique ou physique sur la base de la grandeur de mesure de synchronisation et ensuite, le cas échéant, les évaluer, ou
c) compenser la grandeur de mesure de synchronisation avec l'au moins une grandeur de mesure physiologique ou physique.

10. Procédé de détermination d'au moins un instant de mesure optimal ou d'un intervalle de temps de mesure optimal pour acquérir au moins une grandeur de mesure physiologique ou physique au moyen d'un capteur agencé dans ou sur un bioréacteur, comportant :
- introduire dans le bioréacteur un mouvement de brassage au moyen d'un dispositif de mouvement,
- produire une grandeur de mesure de synchronisation au moyen d'un capteur ou transmetteur destiné à produire une grandeur de mesure de synchronisation,
- et, au moyen d'un dispositif de synchronisation,
a) déclencher la mesure d'au moins une grandeur de mesure physiologique ou physique et le cas échéant afficher, enregistrer et/ou évaluer ensuite au moins une valeur mesurée,
b) compenser la grandeur de mesure de synchronisation avec l'au moins une grandeur de mesure physiologique ou physique, ou
c) sélectionner des valeurs enregistrées de l'au moins une grandeur de mesure physiologique ou physique sur la base de la grandeur de mesure de synchronisation, et le cas échéant les évaluer, ou
d) acquérir au moins une grandeur de mesure physiologique ou physique sur la base de la grandeur de mesure de synchronisation, la sélectionner en fonction de la grandeur de mesure de synchronisation selon une règle prédéterminée et l'utiliser ensuite comme prescription pour la suite de l'acquisition de l'au moins une grandeur de mesure physiologique ou physique.

11. Procédé selon la revendication 9 ou 10, dans lequel, pour acquérir la grandeur de mesure physiologique ou physique, un spectre de fréquences est parcouru, et/ou la grandeur de mesure physiologique ou physique consiste en un spectre de fréquences.

12. Procédé selon la revendication 11, dans lequel le spectre de fréquences est acquis de manière discrète et discontinue en fonction de la grandeur de mesure de synchronisation.

13. Utilisation d'un système selon l'une quelconque des revendications 1 à 8 pour brasser un bioréacteur ou dans un procédé selon l'une quelconque des revendications 11 à 13.

14. Utilisation d'un système ou d'un procédé selon l'une quelconque des revendications précédentes pour la culture de micro-organismes et/ou de cellules.

15. Procédé d'apprentissage ou d'étalonnage d'un système selon l'une quelconque des revendications 1 à 8, comportant en outre dans une phase de test une valeur de seuil concernant la définition ou la détermination de (a) la grandeur de mesure physiologique ou physique ou (b) la grandeur de mesure de synchronisation, valeur de seuil telle que, lorsqu'elle est dépassée par une valeur supérieure ou inférieure, une valeur mesurée, en relation temporelle avec le dépassement par valeur supérieure ou inférieure, de la grandeur de mesure physiologique ou physique est exploitée.
